# EUROPEAN PATENT APPLICATION

(11) **EP 0 248 129 A1**
(43) Date of publication of application: **09.12.1987**
(21) Application number: 86304226.3
(22) Date of filing: 03.06.1986
(51) Int. Cl.: C07C 49/84, C07C 49/813, C07C 45/72, C07C 317/14

(54) **Trihaloacyl aromatic monomers and process for their production**

(71) Applicant: THE DOW CHEMICAL COMPANY, Midland Michigan 48640-1967 (US)
(72) Inventor: St. George, George M., Lake Jackson Texas 77566 (US); Walters, Marlin E., West Columbia Texas 77486 (US)
(74) Representative: Raynor, John

(57) **Abstract**

Adducts of certain trihaloacyl aromatic monomers are described made which are, in themselves, monomers for the preparation of polyesters and polyamides. Such polymers have easier processability than those made with the uncoupled precursor monomers. The monoacylated compound is coupled by reacting with a aldehyde or ketone in the presence of an acid catalyst.

## Description

This invention concerns trihaloacetyl aromatic monomers, useful in preparing polyesters, polyamides and other polymers.

Trihaloacyl derivatives of aromatic ethers, e.g. diphenyl ether, have been found to be useful in preparing polyesters, polyamides and other polymers. The formation of the haloacetyl derivates of aromatic compounds, however, suffers from the drawback of the large amounts of Friedel-Crafts catalyst required. Since the monoacylated diphenyl ether can be made conveniently with substoichiometric quantities of Friedel-Crafts catalyst, it would be advantageous to make bisacylated materials by coupling the monoacylated material, particularly in the present of less expensive catalysts (e.g., H₂SO₄). Even if a stoichiometric quantity of a Friedel-Crafts catalyst is used in the coupling reaction, the net amount of catalyst per bisacylated product would be roughly half that required in the one-step bisacylation. The bisacylated monomers made according to the present process are useful in modifying the properties of polymers made from them.

In U. S. Patent 3,450,772 aromatic halides are reacted with a bisphenol or resorcinol to obtain coupled phenyl ethers. The reaction takes place in the present of an alkali metal hydroxide (to form the alkali metal salt of the phenol) and is catalyzed by a copper salt, e.g. cuprous chloride. The trichloroacetyl products of the present invention could not be made by this reaction, however, because the trichloroacetyl group is more active than the aromatic halogen atom and benzoic acid ester would result instead of a bis(trichloroacetyl) compound.

The present invention concerns a process to obtain bisacylated monomers and the monomer products made thereby.

Adducts of certain trihaloacyl aromatic monomers have now been made which are, in themselves, monomers for the preparation of polyesters and polyamides as well as other polymers which have easy processability. The monoacylated compound is coupled by reacting with a coupling agent in the presence of an acid catalyst, e.g. a strong acid catalyst or a Lewis acid catalyst. The coupled products of the invention, having two trihaloacetyl groups on each molecule, can be reacted with dihydric alcohols and diamines to form polyesters and polyamides, respectively. Other polymers, such as polyurethanes, can also be prepared.

Coupling reactions are employed to produce trihaloacyl derivatives of aromatic compounds of the formula 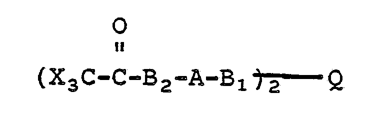 wherein there are more than two aromatic rings between the two halogen-containing functional groups. Thus, two moles of a compound having the structure 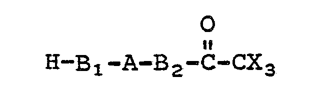
wherein B₁ and B₂ are each independently 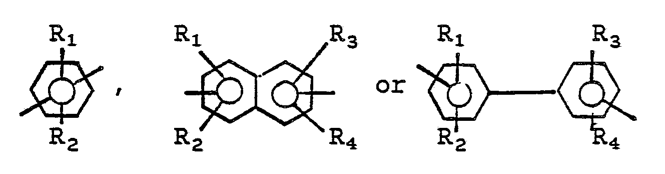 wherein R₁, R₂, R₃ and R₄ are each independently hydrogen, chlorine, bromine, an alkyl or an alkoxy group having from 1 to 4 carbon atoms, a phenyl group or 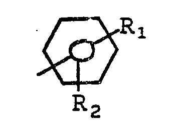 wherein R₁ and R₂ have their previous meanings;
X is chlorine or bromine; 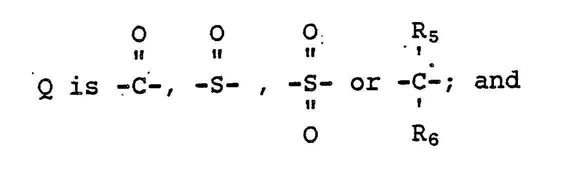 A is a single valence bond, 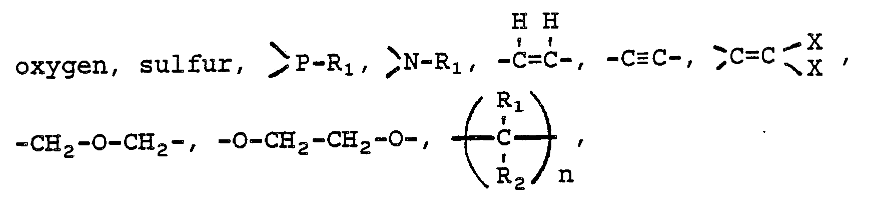 an aromatic group having the formula 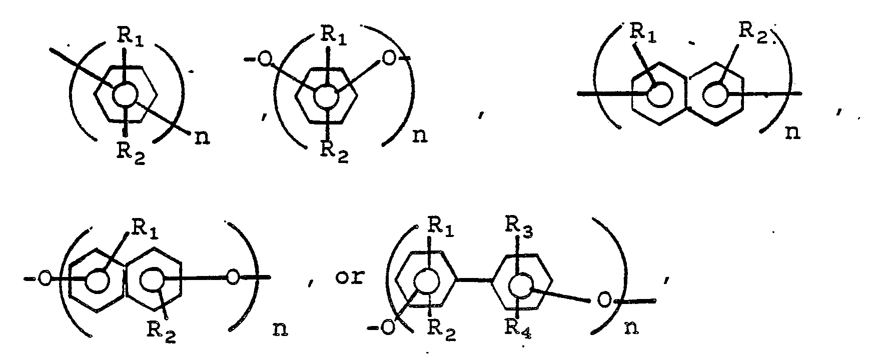 where X, R₁, R₂ R₃ and R₄ have their previous meanings, R₅ and R₆ are each independently hydrogen, halogen, alkyl groups or halosubstituted alkyl groups having 1 to 6 carbon atoms, an aryl group having 6 to 10 carbon atoms or wherein the alkyl groups are joined to form a dihaloalkene or a cyclic ring containing from 3 to 6 carbon atoms and the halogen is chlorine or bromine; and n is an integer from 1 to 6, are reacted with a coupling agent in the presence of an acid catalyst. Preferably B₁ has one less valence bond than B₂.

Examples of aryl groups are aromatic groups having 6 to 10 carbon atoms, such as phenyl, naphthyl, and others.

Representative of aldehydes and ketones, useful as coupling agents when Q is 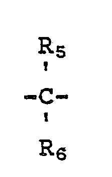 where R₅ and R₆ are defined as before but excluding halogen, are acetaldehyde, propionaldehyde, chloral, bromal, formaldehyde, benzaldehyde, acetone, acetophenone, and cyclohexanone.

Other coupling agents, having the structure QX₂ wherein X is defined as above and Q is 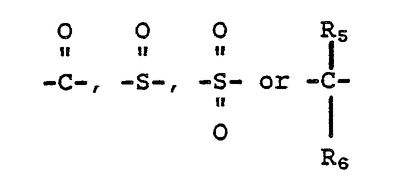 wherein R₅ and R₆ are each independently hydrogen, halogen, an alkyl or haloalkyl group having from 1 to 6 carbon atoms or an aryl group having from 6 to 10 carbon atoms, are used when the acid catalyst is a Lewis acid.

Where both R₅ and R₆ are halogens they may be hydrolyzed to form the carbonyl group, thus yielding the product having the following formula 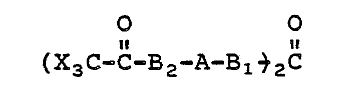 wherein A, B₁, B₂ and X have their previous meanings.

The acid catalyst for the coupling reaction, when Q is 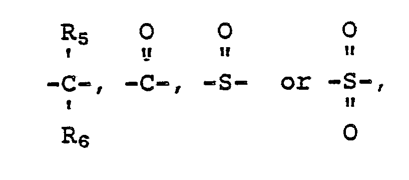 may be any Lewis acid-type compound which will catalyze the Friedel-Crafts reaction. Compounds useful are, for example, AlCl₃, ZrCl₄, TiCl₄, TiCl₃, TaF₅, and HfCl₄.

The acid catalyst for the coupling reaction, when Q is 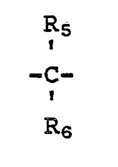 where R₅ and R₆ are defined as before but excluding halogen, may be any strong acid compound. Thus, H₂SO₄, CF₃SO₃H, FSO₃H, HCl, H₃PO₄ and HBF₄ can be used; also, organic sulfonic acids, e.g., toluenesulfonic acid and strong acid ion exchange resins can be employed.

This reaction is conducted at temperatures in the range of from about 20° to about 150°C and preferably from about 45°C to about 80°C.

Solvents for the coupling reaction include chlorinated hydrocarbons, e.g., chloroform, methylene chloride, carbon tetrachloride or tetrabromide, 1,1-dichloroethane, phosgene, dibromocarbonyl, tetrachloroethanes, tetrabromoethanes, 1,1-dichloropropane, or 2,2-dichloropropane. The selection of solvent will depend upon the range of temperature needed for the reaction. Also the solvent must not adversely affect or be affected by the catalyst. Thus, methylene chloride can be used as a coupling agent and solvent.

The following experiments are representative of the process and products of the present invention. Preparation of the singly trichloroacetylated diphenyl ether is shown in Example A. This is a representative starting material for preparing the coupled products of the invention.

### EXAMPLE A

In a typical synthesis of 4-trichloroacetyl diphenyl ether, a 500 ml round-bottom flask fitted with a condenser, thermometer, and pressure-equalizing dropping funnel was charged with 200 ml trichloroacetyl chloride, (TCAC, 1.8 moles) and 15 g AlCl₃ (0.11 moles). The funnel was charged with 80 ml diphenyl ether (0.50 moles). The system was purged with nitrogen, and the diphenyl ether was added slowly to the AlCl₃/TCAC slurry at room temperature with stirring. The temperature was then raised to 85°C. After 2 hours, gas chromatography showed only the monosubstituted diphenyl ether, with no diphenyl ether remaining and little or no 4,4ʹ-bis-(trichloroacetyl)phenyl ether. The resulting mixture was quenched in ice/HCl.

As the mixture approached room temperature, the rate of the hydrolysis of the excess TCAC became more rapid and its exothermic nature raised the temperature more rapidly. Sufficient time was allowed for completion of hydrolysis and for the reaction mixture to return to room temperature.

The product was extracted with CH₂Cl₂. The organic phase was washed with water, dried with Na₂SO₄, and stripped on a rotary evaporator. The product was removed from dark impurities by distillation at 2 torr and 180°C to yield 110 g of a colorless or pale yellow oil (0.35 moles, 70 percent). The oil solidified on standing to form crystals, m.p. 46-48°C.

In the following examples the coupling of the product of Example A is shown.

### EXAMPLE 1

A quantity of 4-(trichloracetyl)diphenyl ether, 12.0 g (0.038 mole) was dissolved in 20 ml CHCl₃ in a 100 ml round-bottom flask fitted with a reflux condenser and containing a stirring bar. While the solution was stirred, 10 ml H₂SO₄ (concentrated, 0.18 mole) was added. To this (now dark) solution was added an excess of chloral (4 ml, 0.041 mole). The solution was brought to reflux; and the reaction was continued until all of the starting ether was used (about 36 hrs), as monitored by gas chromatographic means. As necessary, CHCl₃ was added to maintain stirring. The final solution was poured into a large beaker containing 100 ml H₂O. To this milky emulsion were added 100 ml CH₂Cl₂ and 100 ml CH₃OH. After vigorous stirring, the mixture was poured into a separatory funnel, and the heavier organic layer was recovered. The aqueous layer was extracted three times with 100 ml portions of CH₂Cl₂. All of the organic layers were combined, dried with Na₂SO₄ and filtered. Volatiles were removed on a rotary evaporator, leaving an off-white solid. The yield was 11.7 g, 81 percent.

The product can be crystallized with difficulty by precipitation from a concentrated acetone solution with methanol or ethanol to give a white powder, m.p. 158-162°C. The 1,1,-bis{4-[4-(trichloroacetyl)phenoxy]phenyl}-2,2,2-trichloroethane has the structure

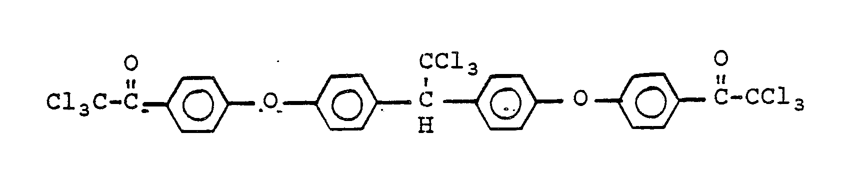

### EXAMPLE 2

A 250 ml three-neck flask fitted with a nitrogen inlet and a condenser was charged with 4-trichloroacetyl diphenyl ether (20 g, 63 mmol); trioxane (2 g, 67 mmol HCHO equivalent); and CHCl₃ (100 ml). FSO₃H (2.3 ml, 40 mmol) was added, and the mixture refluxed under N₂ for three days. Workup in the same manner as for the chloral-coupled product of Example 1 gave primarily 4,4ʹ-bis[4-(trichloroacetyl)phenoxy]diphenylmethane, with some of the 2,4ʹ-isomer, as a light-brown oil (17.6 g, 27 mmol, 87 percent yield).

The compound has the following structure:

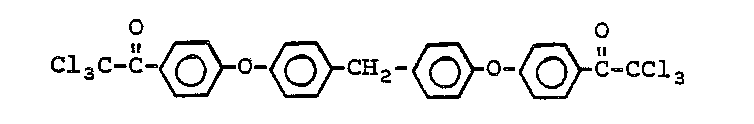

### EXAMPLE 3

A 250 ml three-neck flask fitted with a nitrogen inlet and a condenser was charged with 4-trichloroacetyl diphenyl ether (20 g, 63 mmol); bromal (6.5 ml, 62 mmol); and CHCl₃ (100 ml). FSO₃H (4 ml, 61 mmol) was added and the mixture refluxed under N₂ 36 hours. Workup was as for the chloral-coupled product of Example 1. During reaction and/or workup, the product was dehydrobrominated giving 1,1-dibromo-2,2-bis{4-[4-(trichloroacetyl)phenoxy]phenyl}ethene as a light brown oil (13.6 g, 16.7 mmol, 53 percent). The product has the following structure:

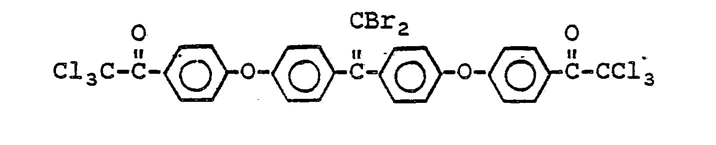

### EXAMPLE 4

In a 500 ml round-bottom flask were combined 31 g 4-(trichloroacetyl)diphenyl ether (0.098 mol); 200 ml CCl₄; and 13 g AlCl₃ (0.098 mol). A condenser was fitted to the flask, and the mixture was heated with vigorous stirring at 60°C for 3 hours. During this time, a dark red, viscous gum separated from the CCl₄. This was taken up into the minimum amount of CH₂Cl₂ (about 200 ml) and the AlCl₃ was quenched by pouring the CH₂Cl₂ solution over 300 g ice and stirring vigorously. On standing a few minutes, two phases separated, the organic phase was dried, and the CH₂Cl₂ was removed, leaving 33.2 g of a dark brown oil (0.047 mol, 94 percent crude yield). For formation of the ketone derivative (see Example 2) no further purification is necessary. The product, bis-{4-[4-(trichloroacetyl)phenoxy]phenyl}dichloromethane has the structure

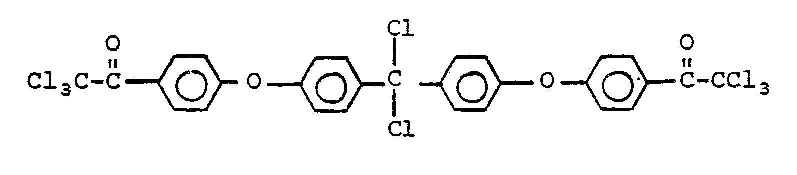

Similar products can be made using 1,1-dichloroethane or 1,1,2-trichloroethane.

### Example 5

A 250 ml round-bottom flask was charged with the product of Example 1, 29.6 g (0.042 mol); 150 ml THF; and 20 ml 0.1N HCl. The flask was fitted with a condenser, and the mixture was stirred at reflux for 2 hours. Upon standing and cooling, two phases separated. The organic phase was dried, and the volatiles were removed to give 28.2 g of a tan-colored gum. Precipitation from acetone and alcohol gave 21.6 g of a white powder (0.033 mols, 79 percent), m.p. 125°C (uncorrected). The product, bis-{4-[4ʹ-(trichloroacetyl)phenoxy]phenyl}methanone, has the structure

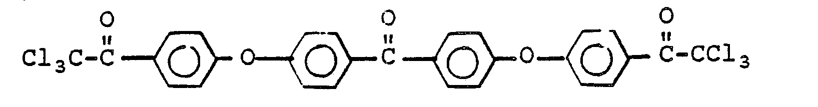

Acetone, dimethyl sulfoxide or dimethyl formamide also can be used as the solvent in the above hydrolysis reaction.

The above compound can also be made directly by reacting the monohaloacylated compound with phosgene or dibromocarbonyl.

### Example 6

A 500-ml, three-neck flask, fitted with a thermometer, a stirring bar, a condenser, a pressure-equalizing dropping funnel, a nitrogen inlet, and a gas outlet, was charged with 200 ml 1,2-dichloroethane; 4-(trichloroacetyl)diphenyl ether (31 g, 98 mmol); and AlCl₃ (20g, 150 mmol). A solution of SOCl₂ (7 ml, 96 mmol) in 1,2-dichloroethane (33 ml) was added dropwise via the dropping funnel to the rapidly stirred mixture under nitrogen. The mixture was heated at 65°C for 2.5 hours, at which time no remaining 4-(trichloroacetyl)diphenyl ether was observed by gas chromatography. The mixture was quenched in ice/HCl. The heavier, organic layer was separated, washed twice with water, and dried with MgSO₄. The volatiles were removed by rotary evaporation to give a yellow oil (39 g). Chromatography on a large (9 x 72 cm) Kieselguhr "dry" column developed with 1:3 ethyl acetate:hexane gave bis-{4-[4ʹ-(trichloroacetyl)phenoxy]phenyl}-sulfoxide (13.7 g, 20 mmol, 41 percent). Evaporation of a 3:1 haxane:CH₂Cl₂ solution gave long, pale-yellow needles, m.p. 154.5-156°C of the structure. 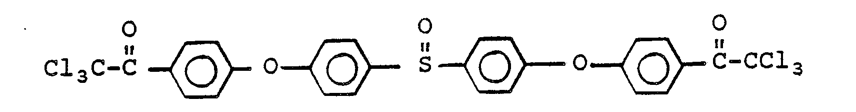 nmr (CDCl₃): δ8.23 (d, 4H, J=8.5 Hz), 7.67 (d, 4H, J=8.5 Hz) 7.16 (d, 4H, J=8.5 Hz), 7.02 (d, 4H, J=8.5 Hz) and
IR (CCl₄): 1713 cm⁻¹ (ν_{co}), 1068 (νₛₒ).

### Example 7

Reaction apparatus was the same as that used in Example 6. The flask was charged with 1,2-dichloroethane (150 ml); 4-(trichloroacetyl)diphenyl ether (31 g, 98 mmol); and AlCl₃ (40 g, 300 mmol). SO₂Cl₂ (25 ml, 310 mmol) was added via the dropping funnel. The mixture was heated at 70°C until all of the 4-(trichloroacetyl)diphenyl ether was consumed (2 hours). Quenching, washing, and drying were performed as per Example 6. Removal of the volatiles gave 41.2 g of a dark-green gum. Separation by elution through a Kieselguhr column with increasingly polar ethyl acetate-hexane mixtures gave 1-chloro-4-[4ʹ-(trichloroacetyl)phenoxy]benzene as a major product, with a smaller amount of bis-{4-[4ʹ-(trichloroacetyl)phenoxy]phenyl}sulfone (2.7 g, 4 mmol, 8 percent) as a light-green oil of the structure 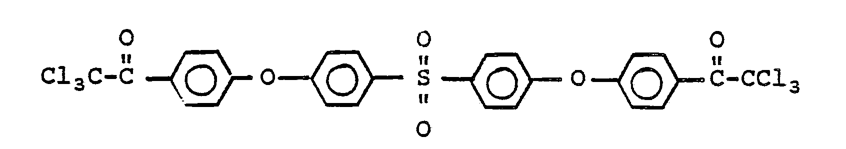 nmr (CDCl₃):δ8.22 (d, 4H, J=9.0 Hz), 7.95 (d, 4H, J=9.0 Hz), 7.15 (d, 4H, J=9.0 Hz), 7.07 (d, 4H, J=9.0 Hz) and
IR (CCl₄): 1705 cm⁻¹ (ν_{co}), 1382 (νₛₒ), 1168 (νₛₒ).

The dimeric, coupled trihaloacetyl diphenyl ether compounds of the above examples and similar compounds conforming to the structure 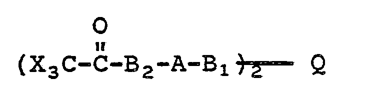 wherein A, B₁, B₂, R₅, R₆, Q and X have the meaning prescribed before, can be used to make polymers, e.g. polyesters by reacting with a dihydric alcohol.

Representative of the dihydric alcohols for the polyester reaction are bisphenol A (p,pʹ-isopropylidene diphenol), resorcinol, hydroquinone, [1,1ʹ-biphenyl]-4,4ʹ-diol, 1,5-dihydroxynaphthalene, 1,1-dichloro-2,2-bis(4-hydroxyphenyl)ethylene, 1-(4-hydroxyphenyl)-1,3,3-trimethyl-7-hydroxyindane, 4,4ʹ-dihydroxyphenyl ether, 1,4-dihydroxy anthraquinone, 3,3ʹ,5,5ʹ-tetramethyl-4,4ʹ- dihydroxybiphenyl, 1,4-cyclohexanediol; also alkylene glycols, e.g., ethylene and propylene glycols, 1,4-butane-diol and polyols.

When reacted with diamines such coupled compounds will form polyamides. Examples of diamines useful in making the polyamides are ethylene diamine, 1,6-diaminohexane, propylene diamine, 4,4ʹ-diaminobiphenyl, p-phenylenediamine, m-phenylenediamine, (toluenediamine)2,4-diaminoaminophenyl sulfone (diaminodiphenylsulfone), 4,4ʹ-diaminodiphenylamine, 4,4ʹ-diaminophenyl ether, 1,6-hexanediamine, 1,4-cyclohexanediamine, 4,4ʹ-trimethylenedipiperidine and bis(amino)polyglycols.

The reaction to make the polyester and polyamides is generally conducted in the presence of a basic catalyst. If, however, the reactant amine is sufficiently basic, no catalyst is required.

While the reaction can be conducted in a melt of the reactants, a polar aprotic solvent is frequently used to advantage. Representative of such solvents useful in the reaction include tetrahydrofuran (THF), sulfolane, γ-butyrolactone, dimethylsulfoxide (DMSO), dimethylacetaminde, dimethylformamide (DMF), and N-methylpyrrolidone.

The catalysts useful in the reaction are basic catalysts. Alkali metal hydrides, e.g. NaH, can be employed and also tertiary amines, such as trialkyl amines, e.g. triethyl amine. Lithium alkyls and amides, e.g. butyl lithium and diisopropyl lithium amide, are also good catalysts for the reaction.

The polymerization reaction may be conducted over a broad temperature range from about -100°C to about +200°C, preferably from about -45° to about +85°C. One of the advantages of this process for making polymers is the low temperature at which it may be conducted, which avoids decomposition which can occur at higher temperatures.

The time for the polymerization to take place will vary with the temperature and with the relative reactivities of the reactant alcohols, amines, and trichloroacetyl derivatives. The reaction is generally accomplished in from about 1 minute to 24 hours and preferably from about 6 minutes to about 12 hours. The reaction is normally very rapid at room temperatures and requires less than an hour, usually only a few minutes.

Pressures may be employed within the range of from about 20 to 760 mm Hg. Higher pressure can be employed, but is of no particular advantage. The pressure employed is not a critical variable.

With respect to the polymers made from the bis(trichloroacetyl) compounds of this invention, such monomers, having different linkages incorporated into the backbone of the polymer, would produce different properties, e.g. flexibility. The halogen substituted ketones and aldehydes used for the coupling would provide the polymer with fire retarding properties.

## Claims

1. A bis(haloacylaromatic) monomers of the formula 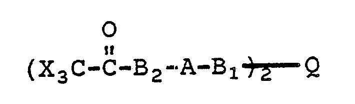 wherein B₁ and B₂ are each independently 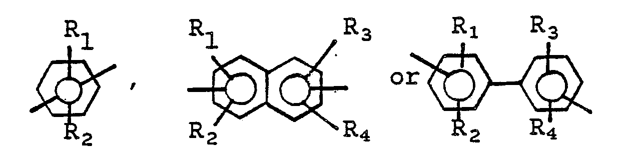 wherein R₁, R₂, R₃ and R₄ are each independently hydrogen, chlorine, bromine, an alkyl or an alkoxy group having from 1 to 4 carbon atoms, a phenyl group or 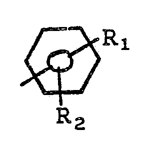 wherein R₁ and R₂ have their previous meanings;
X is chlorine or bromine; 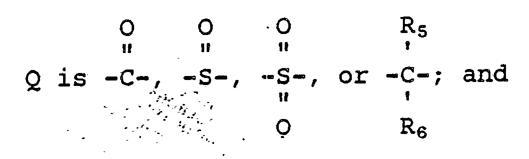 A is a single valence bond, oxygen, sulfur, 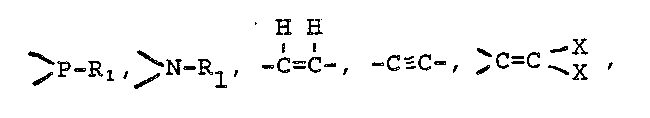 -CH₂-O-CH₂-, -O-CH₂-CH₂-O-, 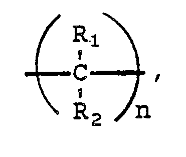 an aromatic group having the formula 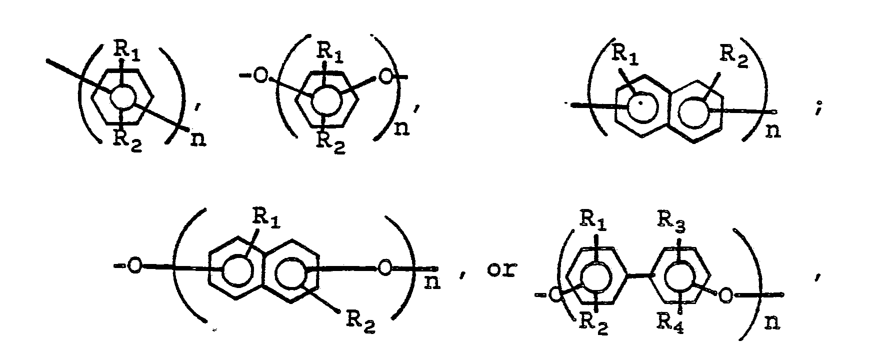 where X, R₁, R₂, R₃ and R₄ have their previous meanings; R₅ and R₆ are each independently hydrogen, halogen, alkyl groups or halosubstituted alkyl groups having 1 to 6 carbon atoms, an aryl group having 6 to 10 carbon atoms or wherein the alkyl groups are joined to form a dihaloalkene or a cyclic ring containing from 3 to 6 carbon atoms and the halogen is chlorine or bromine; and n is an integer from 1 to 6.

2. The monomer of Claim 1 wherein X is chlorine, R₁, R₂, R₃, R₄ and R₅ are each hydrogen, and A is oxygen.

3. The monomer of Claim 2 wherein both B₁ and B₂ are a phenyl group.

4. 1,1-bis-{4-[4-trichloroacetyl)phenoxy]phenyl}-2,2,2,-trichloroethane,
4,4ʹ-bis[4-(trichloroacetyl)phenoxy]diphenylmethane,
1,1-dibromo-2,2-bis{4-[4-(trichloroacetyl)phenoxy]phenyl}ethene,
bis{4-[4ʹ-(trichloroacetyl)phenoxy]phenyl}dichloromethane,
bis{4-[4ʹ-(trichloroacetyl)phenoxy]phenyl}methanone,
bis{4-[4ʹ-(trichloroacetyl)phenoxy]phenyl}sulfoxide, or
bis{4-[4ʹ-(trichloroacetyl)phenoxy]phenyl}sulfone.

5. A process for preparing the bis(haloacylaromatic) monomers of Claim 1 which comprises reacting a compound of the formula 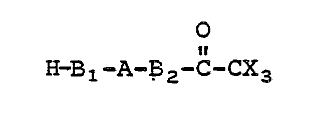
wherein A, B₁, B₂, and X are defined as in Claim 1, R₅ and R₆ are each independently hydrogen, halogen, alkyl groups or halosubstituted alkyl groups having 1 to 6 carbon atoms or an aryl group having 6 to 10 carbon atoms or wherein the alkyl groups are joined to form a dihaloalkene or a cyclic ring containing from 3 to 6 carbon atoms and the halogen is chlorine or bromine, with a coupling agent in the presence of an acid catalyst.

6. The process of Claim 5 wherein a chlorinated hydrocarbon is used as a solvent.

7. The process of Claim 5 or 6 wherein the reaction is conducted at a temperature of from 20 to 150°C.

8. The process of any one of claims 5 to 7 wherein the acid catalyst is a strong mineral acid and Q is 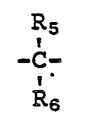 where R₅ and R₆ are each independently hydrogen, alkyl groups or halosubstituted alkyl groups having 1 to 6 carbon atoms or an aryl group having 6 to 10 carbon atoms or wherein the alkyl groups are joined to form a dihaloalkene or a cyclic ring containing from 3 to 6 carbon atoms and the halogen is chlorine or bromine.

9. The process of Claim 8 wherein the mineral acid is H₂SO₄, or fluorosulfonic acid.

10. The process of Claim 5 wherein the catalyst is a strong acid ion exchange resin.

11. The process of Claim 5 wherein the catalyst is fluorosulfonic acid.

12. The process of Claim 5 wherein the coupling agent is an aldehyde or ketone.

13. The process of Claim 5 or 7 wherein the acid catalyst is a Lewis acid and Q is

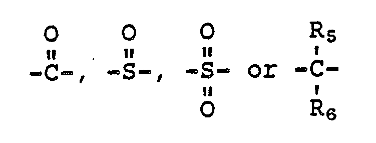

14. The process of Claim 13 wherein the Lewis acid is AlCl₃.

15. The process of Claim 5 or 14 wherein the coupling agent is methylene chloride.
